(19) Europäisches Patentamt
European Patent Office
Office européen des brevets

(11) Publication number: 0 070 562 B1

# (12) EUROPEAN PATENT SPECIFICATION

(45) Date of publication of patent specification: 12.06.85

(51) Int. Cl.[4]: C 07 D 457/02, C 07 D 457/06, A 61 K 31/48

(21) Application number: 82106539.8

(22) Date of filing: 20.07.82

## (54) Ergoline derivatives, processes for their preparation and pharmaceutical compositions containing them.

(30) Priority: 21.07.81 GB 8122356
31.03.82 GB 8209544

(43) Date of publication of application:
26.01.83 Bulletin 83/04

(45) Publication of the grant of the patent:
12.06.85 Bulletin 85/24

(84) Designated Contracting States:
AT BE CH DE FR GB IT LI NL SE

(56) References cited:
GB-A-1 512 874
GB-A-2 058 746

(73) Proprietor: FARMITALIA CARLO ERBA S.p.A.
Via Carlo Imbonati n.24
I-20159 Milan (IT)

(72) Inventor: Luigi, Bernardi
Via Pinerolo 30
Milan (IT)
Inventor: Temperilli, Aldemio
Via Anguissola, 21
Milan (IT)
Inventor: Bosisio, Germano
Via De Santis, 23/B
Palazzolo Milanese (Paderno Dugnano)MI (IT)
Inventor: Traquandi, Gabriella
Via XXV Aprile, 29
Cornate D'adda (Milan) (IT)
Inventor: Eccel, Rosanna
Via XXV Aprile, 29
Cornate D'adda (Milan) (IT)
Inventor: Rossi, Alessandro
Via L. Barzini, 7
Milan (IT)
Inventor: Salvati, Patricia
Via Valera, 16
Arese, Milan (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 070 562 B1

Courier Press, Leamington Spa, England.

(74) Representative: **Hansen, Bernd, Dr.rer.nat. et al**
**Hoffmann, Eitle & Partner Patentanwälte**
**Arabellastrasse 4**
**D-8000 München 81 (DE)**

**Description**

The invention relates to ergoline derivatives, to processes for their preparation, and to pharmaceutical compositions containing them.

The ergoline derivatives according to this invention are having the general formula (I)

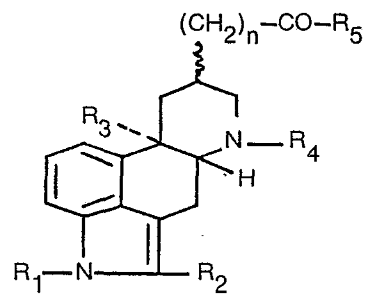

(I)

wherein n is 0, 1 or 2, $R_1$ represents a hydrogen atom or a methyl group, $R_2$ represents a hydrogen or halogen atom or a methyl group, $R_3$ represents a hydrogen atom or a methoxy group, $R_4$ represents a hydrocarbon group having from 1 to 4 carbon atoms, and $R_5$ is a residue of the general formula (II), (III), (IV), (V), (VI), (VII) or (VIII)

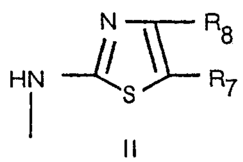

II

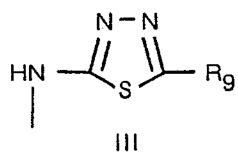

III

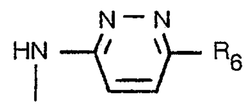

IV

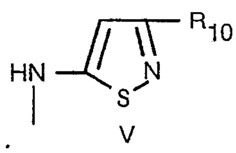

V

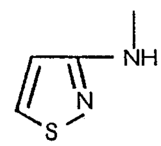

VI

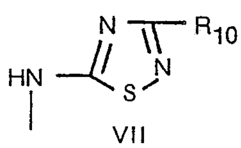

VII

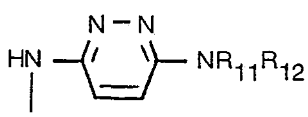

VIII

wherein $R_6$ represents a hydrogen or chlorine atom or a methoxy group, and each of $R_7$, $R_8$ and $R_9$ independently represents a hydrogen atom or an alkyl group having from 1 to 3 carbon atoms, with the proviso that if $R_5$ represents a residue of the general formula (II) and $R_2$, $R_7$ and $R_8$ all represent hydrogen atoms then either $R_4$ does not represent a methyl group or n is not 0, or substituent on $C_8$ is not in β-configuration, and $R_{10}$ represents a hydrogen atom or an alkyl group having from 1 to 3 carbon atoms, and either each of $R_{11}$ and $R_{12}$ independently represents a hydrogen atom, a methyl group or a phenyl group, or $R_{11}$ and $R_{12}$ together with the nitrogen atom to which they are bound form a pyrrolidine, piperidine, morpholine, pyrazole, imidazole or pyrrole ring, and pharmaceutically acceptable salts thereof.

In the general formula the term "halogen" should be construed to preferably encompass chlorine and bromine atom; nevertheless, term "halogen" also encompasses fluorine atom. In the definition of $R_4$, a hydrocarbon group having from 1 to 4 carbon atoms is intended to include alkyl, cycloalkyl and unsaturated (both ethylenically and acetylenically) groups.

Representative moieties include methyl, ethyl, n-propyl, isopropyl, butyl, t-butyl- isobutyl, methylcyclopropyl, allyl and propargyl.

When $R_{11}$ and $R_{12}$ together with the nitrogen atom to which they are bonded form a heterocyclic ring, this ring is preferably selected from the group consisting of pyrrolidine, piperidine, morpholine, pyrazole, imidazole and pyrrole.

Particularly preferred compounds according to this invention are indicated hereinafter:

6-methyl-8β-(6-chloro-3-pyridazinyl-carbamoyl)-ergoline,

6-methyl-8β-(6-methoxy-3-pyridazinyl-carbamoyl)-ergoline,

1,6-dimethyl-10-methoxy-8β-(6-chloro-3-pyridazinyl-carbamoyl)-ergoline,

2,6-dimethyl-8β-(6-chloro-3-pyridazinyl-carbamoyl)-ergoline,
6-allyl-8β-(6-chloro-3-pyridazinyl-carbamoyl)-ergoline,
6-allyl-8β-(2-thiazolyl-carbamoyl)-ergoline,
6-propyl-8β-(2-thiazolyl-carbamoyl)-ergoline,
6-isopropyl-8β-(2-thiazolyl-carbamoyl)-ergoline,
2,6-dimethyl-8β-(2-thiazolyl-carbamoyl)-ergoline,
6-methyl-8β-[2-(5-methyl-1,3,4-thiadiazolyl)-carbamoyl]-ergoline,
1,6-dimethyl-10-methoxy-8β-[2-(5-methyl-1,3,4-thiadiazolyl)-carbamoyl]-ergoline,
1,6-dimethyl-8β-[2-(5-methyl-1,3,4-thiadiazolyl)-carbamoyl]-ergoline,
6-propyl-8β-[2-(5-methyl-1,3,4-thiadiazolyl)-carbamoyl]-ergoline,
6-allyl-8β-[2-(5-methyl-1,3,4-thiadiazolyl)-carbamoyl]-ergoline,
6-isopropyl-8β-[2-(5-methyl-1,3,4-thiadiazolyl)-carbamoyl]-ergoline,
2,6-dimethyl-8β-[2-(5-methyl-1,3,4-thiadiazolyl)-carbamoyl]-ergoline,
6-methyl-8β-(2-thiazolyl-carbamoylmethyl)-ergoline,
6-methyl-8β-[2-(2-thiazolyl-carbamoyl)-ethyl]-ergoline,
6-methyl-8β-(6-chloro-3-pyridazinyl-carbamoylmethyl)-ergoline,
6-methyl-8β-[2-(6-chloro-3-pyridazinyl-carbamoyl)-ethyl]-ergoline,
6-methyl-8α-[2-(5-methyl-1,3,4-thiadiazolyl)-carbamoyl]-ergoline,
6-methyl-8α-(2-thiazolyl-carbamoyl)-ergoline,
6-methyl-8α-(6-chloro-3-pyridazinyl-carbamoyl)-ergoline,
1-methyl-6-propyl-8β-(2-thiazolyl-carbamoyl)-ergoline,
6-methyl-8β-(6-pyrrolidinyl-3-pyridazinyl-carbamoylmethyl)-ergoline,
6-methyl-8β-[2-(5-methyl-1,3,4-thiadiazolyl)-carbamoylmethyl]-ergoline,
6-methyl-8β-{2-[2-(5-methyl-1,3,4-thiadiazolyl)-carbamoyl]-ethyl}-ergoline,
6-methyl-8β-(3-methyl-5-isothiazolyl-carbamoyl)-ergoline,
6-methyl-8β-(3-isothiazolyl-carbamoyl)-ergoline,
6-methyl-8β-(3-methyl-5-isothiazolyl-carbamoylmethyl)-ergoline,
6-methyl-8β-[2-(3-methyl-5-isothiazolyl-carbamoyl)ethyl]-ergoline,
6-methyl-8β-[2-(3-isothiazolyl-carbamoyl)ethyl]-ergoline,
6-methyl-8β-[5-(3-methyl-1,2,4-thiadiazolyl)-carbamoyl]-ergoline,
6-methyl-8β-[5-(3-methyl-1,2,4-thiadiazolyl)-carbamoylmethyl]-ergoline,
6-methyl-8β-{2-[5-(3-methyl-1,2,4-thiadiazolyl)-carbamoyl]-ethyl}-ergoline,
6-methyl-8β-(3-pyridazinyl-carbamoylmethyl)-ergoline,
6-methyl-8β-(6-methoxy-3-pyridazinyl-carbamoylmethyl)-ergoline,
6-methyl-8β-(6-methylamino-3-pyridazinyl-carbamoylmethyl)-ergoline,
6-methyl-8β-[6-(pyrrolyl)-3-pyridazinyl-carbamoylmethyl]-ergoline,
6-methyl-8β-(3-pyridazinyl-carbamoyl)-ergoline,
6-methyl-8β-[2-(3-pyridazinyl-carbamoyl)-ethyl]-ergoline,
6-methyl-8β-[2-(6-methoxy-3-pyridazinylcarbamoyl)-ethyl]-ergoline,
6-methyl-8β-(6-methylamino-3-pyridazinylcarbamoyl)-ergoline,
6-methyl-8ß-[2-(6-methylamino-3-pyridazinylcarbamoyl)ethyl]-ergoline,
6-methyl-8β-{2-[6-(1-pyrrolyl)-3-pyridazinylcarbamoyl]-ethyl}-ergoline;
6-methyl-8β-[6-(1-pyrrolyl)-3-pyridazinylcarbamoyl]-ergoline.

The ergoline derivatives according to the invention may be prepared by the high-yield mixed anhydride method using the relatively stable and readily accessible mixed anhydride of the appropriate 8-carboxy-ergoline (IX)

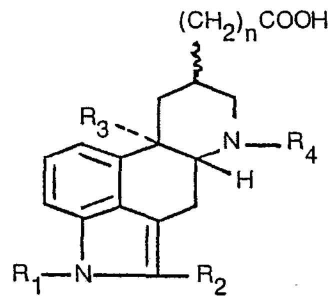

(IX)

wherein n, $R_1$, $R_2$, $R_3$ and $R_4$ have the above meanings, with ethoxyformic acid.

The mixed ethoxyformic anhydride can be prepared by reacting the ergoline (IX) with ethyl chloroformate in a solvent such as tetrahydrofuran, dimethylformamide or dioxan at 0°C for 5—6 hours in the presence of triethylamine.

The so-obtained mixed anhydride may be converted into an ergoline derivative of the general formula (I) by reacting it with an amine $R_5$—H wherein $R_5$ has the above meaning, at room temperature for a period of 2—24 hours. This process is within the scope of the invention.

The crude product can be purified by crystallization, salt formation or by chromatography on a column of neutral aluminium oxide. The ergoline-8-carboxylic acids (IX) are known compounds or can be prepared by establishing procedures.

The ergoline derivatives according to the invention and their pharmaceutically acceptable salts are useful antidepressant agents and they also display from moderate to good antiprolactinic activity and from moderate to good antihypertensive activity.

Accordingly the invention also provides a pharmaceutical composition comprising an ergoline derivative having the general formula (I) or a pharmaceutically acceptable salt thereof in admixture with a pharmaceutically acceptable diluent or carrier.

The antidepressant activity of the ergoline derivatives according to the invention can be predicted by antagonism to reserpine induced blepharospasm and hypothermia.

As a compound disclosed in Belgian Patent No. 841 049 has a structural formula similar to the compounds of this invention applicant has ascertained by the below test that it prevents reserpine induced blepharospasm and hypothermia with $ED_{50}$ values of 7 and 15 mg/kg p.o.. Said compound is 1,6-dimethyl-8β-(2-thiazolyl-carbamoyl)-ergoline, and it is used as reference standard drug, besides the tricyclic antidepressant imipramine, for the compounds of the present invention.

It has been found that the products of the present invention have a surprisingly higher activity as compared with above referred prior art compound, in preventing reserpine induced symptoms with $ED_{50}$ falling in a dose range of from 0.2 to 5 mg/kg p.o., and with very low orientative acute toxicities as the following table shows:

| product of example | reserpine antagonism ($ED_{50}$, mg/kg p.o.) | | orientative acute toxicity ($LD_{50}$, mg/kg p.o.) |
|---|---|---|---|
| | blepharospasm | hypothermia | |
| 7 | 0.5 | 0.6 | >300 |
| 6 | 2.5 | 4.0 | >300 |
| 4 | 0.2 | 0.6 | >300 |
| 16 | 1.0 | 1.7 | >800 |
| 5 | 3.6 | 4.5 | >600 |
| 10 | 3.5 | 3.1 | >600 |
| Imipramine | 8.1 | 18.2 | 200—400 |

The tests were performed in an air-conditioned room at the constant temperature of 19 ± 1°C. Randomized groups of male mice were respectively treated by gavage with appropriate screening doses (from a maximum of 25 mg/kg below) of the test compounds, suspended in the vehicle (methocel 0.5%; 1 ml/100 g b.w.), or with the vehicle alone. One hour later the animals received an i.p. injection of reserpine (1.5 mg/kg; 1 ml/100 g b.w.), and one of the control groups the same volume of the vehicle along (blank controls).

Three hours after pretreatments, i.e. two hours after reserpine or vehicle treatments, blepharospasm was evaluated in scores as proposed by Rubin et al. (J. Pharmacol. *120*, 125 (1957)).

Two hours after the above mentioned evaluation the rectal temperature of the animals was recorded by an appropriate probe connected to a digital thermometer (Ellab DU-3).

The following examples illustrate the invention.

## Example 1

6-methyl-8β-(6-chloro-3-pyridazinyl-carbamoyl)-egoline
(I: $R_1=R_2=R_3=H$, $R_4=CH_3$, $R_5=IV$, $R_6=Cl$)

A mixture of 15.02 g of 6-methyl-8β-carboxy-ergoline and 7 ml of triethylamine in 400 ml of anhydrous tetrahydrofuran was warmed at 50°C for 1 hour. To the cooled suspension 5.34 ml of ethyl chloroformate in 20 ml of tetrahydrofuran were added slowly and the stirring was continued for 6 hours at 0°C. After filtration the clear solution was treated with 7.12 g of 3-amino-6-chloro-pyridazine and left overnight at room temperature. The solution was concentrated under vacuum, dissolved in chloroform and washed with potassium carbonate solution and then with water. The residue of the organic layer was chromatographed through a short column of alumina using chloroform as the eluting solvent and 15 g of the title compound, m.p. 252—254°C, were obtained.

Examples 2 to 38

Operating as in example 1, the compounds in table 2 below were prepared from the following ergoline derivatives and amines;

| Ergoline derivatives | Examples |
|---|---|
| 6-methyl-8β-carboxy-ergoline, | 2, 10, 27, 28, 32 |
| 1,6-dimethyl-10-methoxy-8β-carboxy-ergoline, | 3, 11 |
| 2,6-dimethyl-8β-carboxy-ergoline, | 4, 9, 16 |
| 1,6-dimethyl-8β-carboxy-ergoline, | 12 |
| 6-allyl-8β-carboxy-ergoline, | 5, 6, 14 |
| 6-propyl-8β-carboxy-ergoline, | 7, 13 |
| 6-isopropyl-8β-carboxy-ergoline, | 8, 15 |
| 6-methyl-8β-carboxymethyl-ergoline | 17, 19, 25, 29, 33, 35—38 |
| 6-methyl-8β-(2-carboxyethyl)-ergoline | 18, 20, 26, 30, 31, 34, 40 and 41 |
| 6-methyl-8α-carboxy-ergoline, | 21—23 |
| 1-methyl-6-propyl-8β-carboxy-ergoline | 24 |
| 2-bromo-6-methyl-8β-carboxymethyl-ergoline | 39 |

| Amine | Examples |
|---|---|
| 3-amino-6-methoxy-pyridazine, | 2, 36, 41 |
| 3-amino-6-chloro-pyridazine, | 3—5, 19, 20, 23, 39 |
| 2-amino-thiazole, | 6—9, 17, 18, 22, 24 |
| 2-amino-5-methyl-1,3,4-thiadiazole, | 10—16, 21, 25, 26 |
| 5-amino-3-methyl-isothiazole | 27, 29, 30 |
| 3-amino-isothiazole, | 28, 31 |
| 3-methyl-5-amino-1,2,4-thiadiazole | 32—34 |
| 3-aminopyridazine | 35, 40 |
| 3-amino-6-(1-pyrrolidinyl)-pyridazine, | 37 |
| 3-amino-6-(1-pyrrolyl)-pyridazine, | 38 |

| Example | Product | General Formula I | | | | | | m.p. (°C) | Yield (%) |
|---|---|---|---|---|---|---|---|---|---|
| | | n | $R_1$ | $R_2$ | $R_3$ | $R_4$ | $R_5$ | | |
| 2 | 6-methyl-8β-(6-methoxy-3-pyridazinyl-carbamoyl)-ergoline | 0 | H | H | H | $CH_3$ | IV, $R_6 = CH_3O$ | 273–274 | 65 |
| 3 | 1,6-dimethyl-10-methoxy-8β-(6-chloro-3-pyridazinyl-carbamoyl)-ergoline | 0 | $CH_3$ | H | $CH_3O$ | $CH_3$ | IV, $R_6 = Cl$ | 140–142 | 70 |
| 4 | 2,6-dimethyl-8β-(6-chloro-3-pyridazinyl-carbamoyl)-ergoline | 0 | H | $CH_3$ | H | $CH_3$ | IV, $R_6 = Cl$ | 278–280 | 75 |
| 5 | 6-allyl-8β-(6-chloro-3-pyridazinyl-carbamoyl)-ergoline | 0 | H | H | H | allyl | IV, $R_6 = Cl$ | 230–232 | 65 |
| 6 | 6-allyl-8β-(2-thiazolyl-carbamoyl)-ergoline | 0 | H | H | H | allyl | II, $R_7 = R_8 = H$ | 170–172 | 70 |
| 7 | 6-propyl-8β-(2-thiazolyl-carbamoyl)-ergoline | 0 | H | H | H | $n$-$C_3H_7$ | II, $R_7 = R_8 = H$ | 200–202 | 64 |
| 8 | 6-isopropyl-8β-(2-thiazolyl-carbamoyl)-ergoline | 0 | H | H | H | $i$-$C_3H_7$ | II, $R_7 = R_8 = H$ | 252–254 | 55 |
| 9 | 2,6-dimethyl-8β-(2-thiazolyl-carbamoyl)-ergoline | 0 | H | $CH_3$ | H | $CH_3$ | II, $R_7 = R_8 = H$ | 187–189 | 72 |
| 10 | 6-methyl-8β-[2-(5-methyl-1,3,4-thiadiazolyl)-carbamoyl]-ergoline | 0 | H | H | H | $CH_3$ | III, $R_9 = CH_3$ | 263–265 | 71 |
| 11 | 1,6-dimethyl-10-methoxy-8β-[2-(5-methyl-1,3,4-thiadiazolyl)-carbamoyl]-ergoline | 0 | $CH_3$ | H | $CH_3O$ | $CH_3$ | III, $R_9 = CH_3$ | 218–220 | 68 |
| 12 | 1,6-dimethyl-8β-[2-(5-methyl-1,3,4-thiadiazolyl)-carbamoyl]-ergoline | 0 | $CH_3$ | H | H | $CH_3$ | III, $R_9 = CH_3$ | 152–154 | 80 |
| 13 | 6-propyl-8β-[2-(5-methyl-1,3,4-thiadiazolyl)-carbamoyl]-ergoline | 0 | H | H | H | $n$-$C_3H_7$ | III, $R_9 = CH_3$ | 202–204 | 70 |
| 14 | 6-allyl-8β-[2-(5-methyl-1,3,4-thiadiazolyl)-carbamoyl]-ergoline | 0 | H | H | H | allyl | III, $R_9 = CH_3$ | 156–158 | 67 |
| 15 | 6-isopropyl-8β-[2-(5-methyl-1,3,4-thiadiazolyl)-carbamoyl]-ergoline | 0 | H | H | H | $i$-$C_3H_7$ | III, $R_9 = CH_3$ | 194–196 | 73 |

| Example | Product | General Formula I | | | | | | m.p. (°C) | Yield (%) |
|---|---|---|---|---|---|---|---|---|---|
| | | n | $R_1$ | $R_2$ | $R_3$ | $R_4$ | $R_5$ | | |
| 16 | 2,6-dimethyl-8β-[2-(5-methyl-1,3,4-thiadiazolyl)-carbamoyl]-ergoline | 0 | H | $CH_3$ | H | $CH_3$ | III, $R_9=CH_3$ | 258–260 | 70 |
| 17 | 6-methyl-8β-(2-thiazolyl-carbamoylmethyl)-ergoline | 1 | H | H | H | $CH_3$ | II, $R_7=R_8=H$ | 285–287 | 70 |
| 18 | 6-methyl-8β-[2-(2-thiazolyl-carbamoyl)-ethyl]-ergoline | 2 | H | H | H | $CH_3$ | II, $R_7=R_8=H$ | 246–248 | 72 |
| 19 | 6-methyl-8β-(6-chloro-3-pyridazinyl-carbamoylmethyl)-ergoline | 1 | H | H | H | $CH_3$ | IV, $R_6=Cl$ | 285–287 | 68 |
| 20 | 6-methyl-8β-[2-(6-chloro-3-pyridazinyl-carbamoyl)-ethyl]-ergoline | 2 | H | H | H | $CH_3$ | IV, $R_6=Cl$ | 280–282 | 71 |
| 21 | 6-methyl-8α-[2-(5-methyl-1,3,4-(thiadiazolyl)carbamoyl]-ergoline | 0 | H | H | H | $CH_3$ | III, $R_9=CH_3$ | 293–5 | 69 |
| 22 | 6-methyl-8α-(2-thiazolyl-carbamoyl)ergoline | 0 | H | H | H | $CH_3$ | II, $R_7=R_8=H$ | 253–4 | 72 |
| 23 | 6-methyl-8α-(6-chloro-3-pyridazinyl-carbamoyl)ergoline | 0 | H | H | H | $CH_3$ | IV, $R_6=Cl$ | 300 | 58 |
| 24 | 1-methyl-6-propyl-8β-(2-thiazolyl-carbamoyl)-ergoline | 0 | $CH_3$ | H | H | n-$C_3H_7$ | II, $R_7=R_8=H$ | 168–170 | 75 |
| 25 | 6-methyl-8β-[2-(5-methyl-1,3,4-thiadiazolyl)-carbamoylmethyl]-ergoline | 1 | H | H | H | $CH_3$ | III, $R_9=CH_3$ | 300 | 68 |
| 26 | 6-methyl-8β-{2[2-(5-methyl-1,3,4-thiadiazolyl)-carbamoyl]-ethyl}-ergoline | 2 | H | H | H | $CH_3$ | III, $R_9=CH_3$ | 275–277 | 71 |
| 27 | 6-methyl-8β-(3-methyl-5-isothiazolyl-carbamoyl)-ergoline | 0 | H | H | H | $CH_3$ | V, $R_{10}=CH_3$ | 174–175 | 62 |
| 28 | 6-methyl-8β-(3-isothiazolyl-carbamoyl)-ergoline | 0 | H | H | H | $CH_3$ | VI | 138–140 | 62 |
| 29 | 6-methyl-8β-(3-methyl-5-isothiazolyl-carbamoylmethyl)-ergoline | 1 | H | H | H | $CH_3$ | V, $R_{10}=CH_3$ | 138–140 | 65 |

| Example | Product | General Formula I | | | | | | m.p. (°C) | Yield (%) |
|---|---|---|---|---|---|---|---|---|---|
| | | n | $R_1$ | $R_2$ | $R_3$ | $R_4$ | $R_5$ | | |
| 30 | 6-methyl-8β-[2-(3-methyl-5-isothiazolyl-carbamoyl)-ethyl]-ergoline | 2 | H | H | H | $CH_3$ | V, $R_{10}=CH_3$ | 191–192 | 55 |
| 31 | 6-methyl-8β-[2-(3-isothiazolyl-carbamoyl)-ethyl]-ergoline | 2 | H | H | H | $CH_3$ | VI | 202–204 | 64 |
| 32 | 6-methyl-8β-[5-(3-methyl-1,2,4-thiadiazolyl)-carbamoyl]-ergoline | 0 | H | H | H | $CH_3$ | VII, $R_{10}=CH_3$ | 181–182 | 68 |
| 33 | 6-methyl-8β-[5-(3-methyl-1,2,4-thiadiazolyl)-carbamoylmethyl]-ergoline | 1 | H | H | H | $CH_3$ | VII, $R_{10}=CH_3$ | 243–245 | 60 |
| 34 | 6-methyl-8β-{2-[5-(3-methyl-1,2,4-thiadiazolyl)-carbamoyl]-ethyl}-ergoline | 2 | H | H | H | $CH_3$ | VII, $R_{10}=CH_3$ | 145–147 | 65 |
| 35 | 6-methyl-8β-(3-pyridazinyl-carbamoylmethyl)-ergoline | 1 | H | H | H | $CH_3$ | IV, $R_6=H$ | 230–232 | 61 |
| 36 | 6-methyl-8β-(6-methoxy-3-pyridazinyl-carbamoylmethyl)-ergoline | 1 | H | H | H | $CH_3$ | IV, $R_6=OCH_3$ | 258–260 | 71 |
| 37 | 6-methyl-8β-(6-pyrrolidinyl-3-pyridazinyl-carbamoylmethyl)-ergoline | 1 | H | H | H | $CH_3$ | VIII, $R_{11}$ and $R_{12}$= 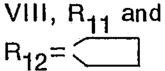 | 263–5 | 67 |
| 38 | 6-methyl-8β-[6-(pyrrolyl)-3-pyridazinyl-carbamoylmethyl]-ergoline | 1 | H | H | H | $CH_3$ | VIII, $R_{11}$ and $R_{12}$= 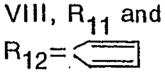 | 241–243 | 70 |
| 39 | 2-bromo-6-methyl-8β-[(6-chloro-3-pyridazinyl)carbamoylmethyl]-ergoline | 1 | H | Br | H | $CH_3$ | IV, $R_6=Cl$ | 254–6 | 65 |
| 40 | 6-methyl-8β-[2(3-pyridazinyl-carbamoyl)ethyl]-ergoline | 2 | H | H | H | $CH_3$ | IV, $R_6=H$ | 233–5 | 75 |
| 41 | 6-methyl-8β-[2(6-methoxy-3-pyridazinyl-carbamoyl)ethyl] ergoline | 2 | H | H | H | $CH_3$ | IV, $R_6=OCH_3$ | 268–270 | 69 |

**Claims for the Contracting States: BE CH DE FR GB IT LI NL SE**

1. Ergoline derivatives having the formula (I)

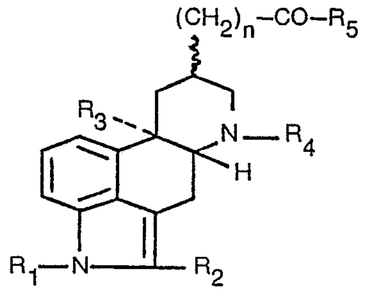

(I)

wherein n is 0, 1 or 2, $R_1$ represents a hydrogen atom or a methyl group, $R_2$ represents a hydrogen or halogen atom or a methyl group, $R_3$ represents a hydrogen atom or a methoxy group, $R_4$ represents a hydrocarbon group having from 1 to 4 carbon atoms, and $R_5$ is a residue of formula (II), (III), (IV), (V), (VI), (VII) or (VIII)

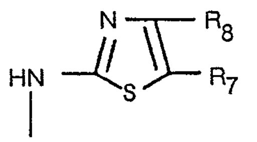

II

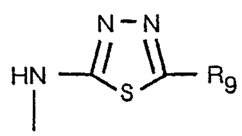

III

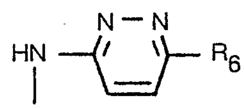

IV

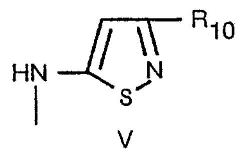

V

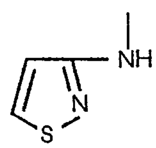

VI

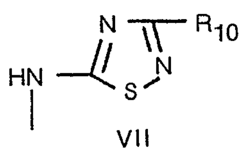

VII

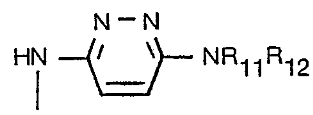

VIII

wherein $R_6$ represents a hydrogen or chlorine atom or a methoxy group, and each of $R_7$, $R_8$ and $R_9$ independently represents a hydrogen atom or an alkyl group having from 1 to 3 carbon atoms, with the proviso that if $R_5$ represents a residue of the general formula (II) and $R_2$, $R_7$ and $R_8$ all represent hydrogen atoms, then either $R_4$ does not represent a methyl group or n is not 0, or substituent on $C_8$ is not in β-configuration and $R_{10}$ represents a hydrogen atom or an alkyl group having from 1 to 3 carbon atoms, and either each of $R_{11}$ and $R_{12}$ independently represents a hydrogen atom, a methyl group of a phenyl group, or $R_{11}$ and $R_{12}$ together with the nitrogen atom to which they are bound form a pyrrolidine, piperidine, morpholine, pyrazole, imidazole or pyrrole ring, and pharmaceutically acceptable salts thereof.

2. A compound according to claim 1, wherein the heterocyclic ring which $R_{11}$ and $R_{12}$ form when they are bonded together, is selected from the group consisting of pyrrolidine, piperidine, morpholine, pyrazole, imidazole and pyrrole.

3. A compound according to claim 1, which is selected from the group consisting of:
6-methyl-8β-(6-chloro-3-pyridazinyl-carbamoyl)-ergoline,
6-methyl-8β-[2-(6-chloro-3-pyridazinyl-carbamoyl)ethyl]-ergoline, and
6-methyl-8β-(6-pyrrolyl-3-pyridazinyl-carbamoylmethyl)-ergoline.

4. A process for preparing an ergoline derivative according to claims 1, 2 or 3, which process comprises reacting a compound of formula (IX)

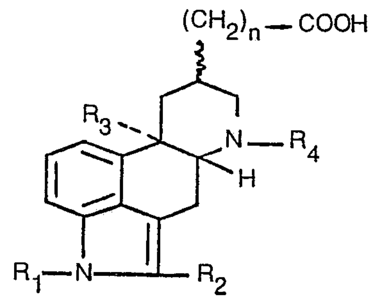

(IX)

wherein n, $R_1$, $R_2$, $R_3$ and $R_4$ are as defined in claim 1, with ethyl chloroformate in an aprotic solvent and condensing the resultant compound with an amine of formula $R_5$—H, wherein $R_5$ is as defined in claim 1.

5. A process according to claim 4, wherein said aprotic solvent is selected from the group consisting of tetrahydrofuran, dimethylformamide and dioxane.

6. A process according to claims 4 or 5, wherein the reaction of the compound of formula (IX) as defined in claim 4 with ethyl chloroformate is carried out at a temperature of 0°C for 5—6 hours in the presence of an organic base.

7. A process according to claim 6, wherein said organic base is triethylamine.

8. A process according to claim 4, wherein said condensation is carried out at room temperature for from 2—24 hours.

9. A pharmaceutical composition containing a therapeutically effective amount of a compound according to anyone of claims 1—3, in admixture with a pharmaceutically acceptable carrier.

10. A pharmaceutical composition according to claim 9 for oral or parenteral administration.

**Claims for the Contracting State: AT**

1. A process for preparing an ergoline derivative having the formula (I)

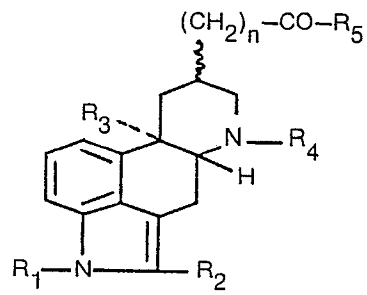

(I)

wherein n is 0, 1 or 2, $R_1$ represents a hydrogen atom or a methyl group, $R_2$ represents a hydrogen or halogen atom or a methyl group, $R_3$ represents a hydrogen atom or a methoxy group, $R_4$ represents a hydrocarbon group having from 1 to 4 carbon atoms, and $R_5$ is a residue of formula (II), (III), (IV), (V), (VI), (VII) or (VIII)

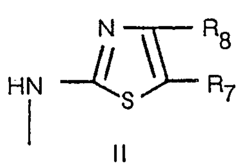

II

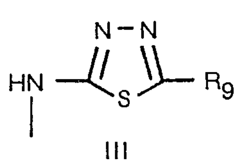

III

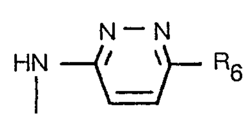

IV

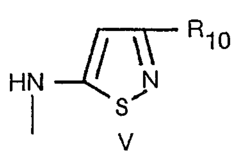

V

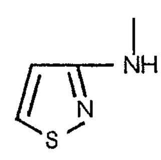

VI

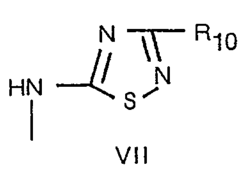

VII

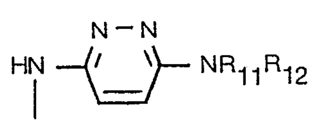

VIII

wherein $R_6$ represents a hydrogen or chlorine atom or a methoxy group, and each of $R_7$, $R_8$ and $R_9$ independently represents a hydrogen atom or an alkyl group having from 1 to 3 carbon atoms, with the proviso that if $R_5$ represents a residue of the general formula (II) and $R_2$, $R_7$ and $R_8$ all represent hydrogen atoms, then either $R_4$ does not represent a methyl group or n is not 0, or substituent on $C_8$ is not in β-configuration and $R_{10}$ represents a hydrogen atom or an alkyl group having from 1 to 3 carbon atoms, and either each of $R_{11}$ and $R_{12}$ independently represents a hydrogen atom, a methyl group or a phenyl group, or $R_{11}$ and $R_{12}$ together with the nitrogen atom to which they are bound form a pyrrolidine, piperidine, morpholine, pyrazole, imidazole or pyrrole ring, and pharmaceutically acceptable salts thereof, which process comprises reacting a compound of the formula (IX)

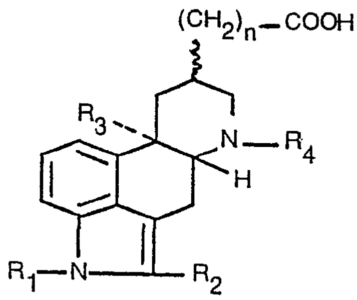

(IX)

wherein n, $R_1$, $R_2$, $R_3$ and $R_4$ are as defined above, with ethyl chloroformate in an aprotic solvent and condensing the resultant compound with an amine of formula $R_5$—H, wherein $R_5$ is as defined above, and if required, converting the resulting compound in a pharmaceutically acceptable acid addition salt in a manner known per se.

2. A process according to claim 1, wherein said aprotic solvent is selected from the group consisting of tetrahydrofuran, dimethylformamide and dioxane.

3. A process according to claims 1 or 2, wherein the reaction of the compound of formula (IX) as defined in claim 1, with ethyl chloroformate is carried out at a temperature of 0°C for 5—6 hours in the presence of an organic base.

4. A process according to claim 3, wherein said organic base is triethylamine.

5. A process according to claim 1, wherein said condensation is carried out at room temperature for from 2 to 24 hours.

**Patentansprüche für die Vertragsstaaten: BE CH DE FR GB IT LI NL SE**

1. Ergolin-Derivate der Formel (I)

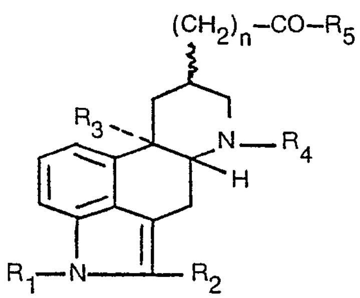

(I)

worin n die Zahl 0, 1 oder 2 darstellt, $R_1$ ein Wasserstoffatom oder eine Methylgruppe bedeutet, $R_2$ ein Wasserstoff- oder Halogenatom oder eine Methylgruppe darstellt, $R_3$ ein Wasserstoffatom oder eine Methoxygruppe bedeutet, $R_4$ eine Kohlenwasserstoffgruppe mit 1 bis 4 Kohlenstoffatomen darstellt, und $R_5$ einen Rest der allgemeinen Formel (II), (III), (IV), (V), (VI), (VII) oder (VIII) bedeutet:

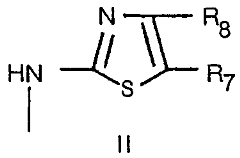

II

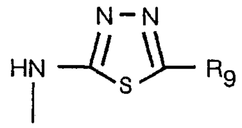

III

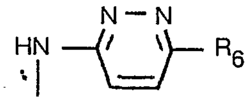

IV

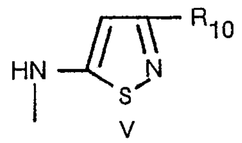

V

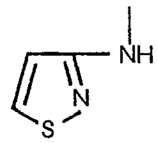

VI

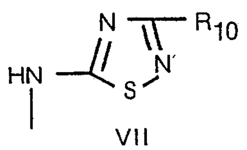

VII

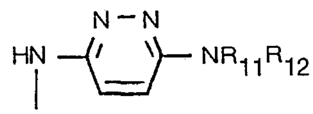

VIII

worin $R_6$ ein Wasserstoff- oder Chloratom oder eine Methoxygruppe bedeutet und $R_7$, $R_8$ und $R_9$ jeweils unabhängig voneinander ein Wasserstoffatom oder eine Alkylgruppe mit 1 bis 3 Kohlenstoffatomen bedeuten, vorausgesetzt, dass wenn $R_5$ einen Rest der allgemeinen Formel (II) darstellt und $R_2$, $R_7$ und $R_8$ sämtliche Wasserstoffatome bedeuten, entweder $R_4$ keine Methylgruppe bedeutet oder n nicht die Zahl 0 darstellt, oder sich der Substituent an $C_8$ nicht in β-Konfiguration befindet, und $R_{10}$ ein Wasserstoffatom oder eine Alkylgruppe mit 1 bis 3 Kohlenstoffatomen bedeutet, und entweder $R_{11}$ und $R_{12}$ jeweils unabhängig voneinander ein Wasserstoffatom, eine Methylgruppe oder eine Phenylgruppe darstellen, oder $R_{11}$ und $R_{12}$ zusammen mit dem Stickstoffatom, an welches sie gebunden sind, einen Pyrrolidin-, Piperidin-, Morpholin-, Pyrazol-, Imidazol- oder Pyrrol-Ring bilden, und pharmazeutisch annehmbare Salze derselben.

2. Verbindung nach Anspruch 1, dadurch gekennzeichnet, dass der heterocyclische Ring, den $R_{11}$ und $R_{12}$ bilden, wenn sie mit einander verbunden sind, ausgewählt wird aus der Gruppe bestehend aus Pyrrolidin, Piperidin, Morpholin, Pyrazol, Imidazol und Pyrrol.

3. Verbindung nach Anspruch 1, dadurch gekennzeichnet, dass sie aus der folgenden Gruppe ausgewählt wird:

6-Methyl-8β-(6-chloro-3-pyridazinyl-carbamoyl)-ergolin,
6-Methyl-8β-[2-(6-chloro-3-pyridazinyl-carbamoyl)-ethyl]-ergolin, und
6-Methyl-8β-(6-pyrrolyl-3-pyridazinyl-carbamoyl-methyl)-ergolin.

4. Verfahren zur Herstellung eines Ergolin-Derivates nach Ansprüchen 1, 2 oder 3, gekennzeichnet durch Umsetzung einer Verbindung der Formel (IX)

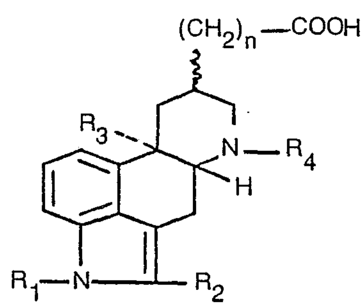

(IX)

worin n, $R_1$, $R_2$, $R_3$ und $R_4$ die im Anspruch 1 angegebene Bedeutung haben, mit Ethylchloroformiat in einem aprotischen Lösungsmittel und Kondensieren der erhaltenen Verbindung mit einem Amin der Formel $R_5$—H, worin $R_5$ die im Anspruch 1 angegebene Bedeutung hat.

5. Verfahren nach Anspruch 4, dadurch gekennzeichnet, dass das genannte aprotische Lösungsmittel ausgewählt wird aus der Gruppe bestehend aus Tetrahydrofuran, Dimethylformamid und Dioxan.

6. Verfahren nach Ansprüchen 4 oder 5, dadurch gekennzeichnet, dass die Reaktion der Verbindung der Formel (IX), wie sie im Anspruch 4 definiert ist, mit Ethylchloroformiat bei einer Temperatur von 0°C während 5 bis 6 h in Gegenwart einer organischen Base durchgeführt wird.

7. Verfahren nach Anspruch 6, dadurch gekennzeichnet, dass die genannte organische Base Triethylamin ist.

8. Verfahren nach Anspruch 4, dadurch gekennzeichnet, dass die genannte Kondensation während 2 bis 24 h bei Raumtemperatur ausgeführt wird.

9. Pharmazeutische Zubereitung, dadurch gekennzeichnet, dass sie eine therapeutisch wirksame Menge einer Verbindung gemäss einem der Ansprüche 1 bis 3 im Gemisch mit einem pharmazeutisch annehmbaren Träger enthält.

10. Pharmazeutische Zubereitung nach Anspruch 9 zur oralen oder parenteralen Verabreichung.

**Patentansprüche für den Vertragsstaat: AT**

1. Verfahren zur Herstellung eines Ergolin-Derivates der Formel (I)

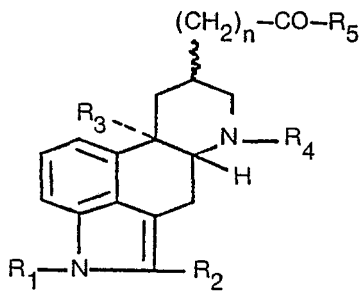

(I)

worin n die Zahl 0, 1 oder 2 darstellt, $R_1$ ein Wasserstoffatom oder eine Methylgruppe bedeutet, $R_2$ ein Wasserstoff- oder Halogenatom oder eine Methylgruppe darstellt, $R_3$ ein Wasserstoffatom oder eine Methoxygruppe bedeutet, $R_4$ eine Kohlenwasserstoffgruppe mit 1 bis 4 Kohlenstoffatomen darstellt, und $R_5$ einen Rest der allgemeinen Formel (II), (III), (IV), (V), (VI), (VII) oder (VIII) bedeutet:

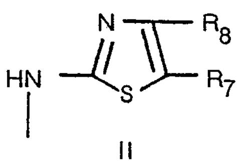

II

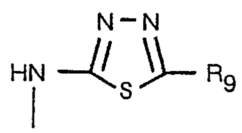

III

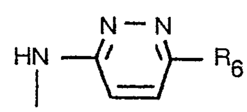

IV

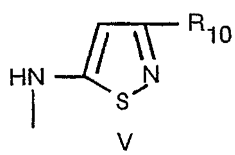

V

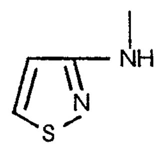

VI

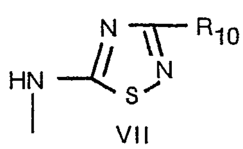

VII

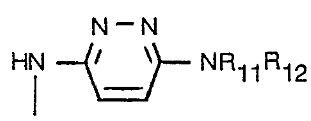

VIII

worin $R_6$ ein Wasserstoff- oder Chloratom oder eine Methoxygruppe bedeutet und $R_7$, $R_8$ und $R_9$ jeweils unabhängig voneinander ein Wasserstoffatom oder eine Alkylgruppe mit 1 bis 3 Kohlenstoffatomen bedeuten, vorausgesetzt, dass wenn $R_5$ einen Rest der allgemeinen Formel (II) darstellt und $R_2$, $R_7$ und $R_8$ sämtliche Wasserstoffatome bedeuten, entweder $R_4$ keine Methylgruppe bedeutet oder n nicht die Zahl 0 darstellt, oder sich der Substituent an $C_8$ nicht in β-Konfiguration befindet, und $R_{10}$ ein Wasserstoffatom oder eine Alkylgruppe mit 1 bis 3 Kohlenstoffatomen bedeutet, und entweder $R_{11}$ und $R_{12}$ jeweils unabhängig voneinander ein Wasserstoffatom, eine Methylgruppe oder eine Phenylgruppe darstellen, oder $R_{11}$ und $R_{12}$ zusammen mit dem Stickstoffatom, an welches sie gebunden sind, einen Pyrrolidin-, Piperidin-, Morpholin-, Pyrazol-, Imidazol- oder Pyrrol-Ring bilden, und pharmazeutisch annehmbare Salze derselben, gekennzeichnet durch Umsetzung einer Verbindung der Formel (IX),

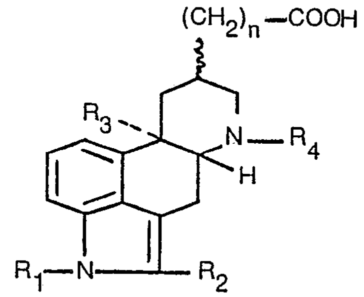

(IX)

worin n, $R_1$, $R_2$, $R_3$ und $R_4$ die vorstehend angebenen Bedeutungen haben, mit Ethylchloroformiat in einem aprotischen Lösungsmittel, und Kondensieren der erhaltenen Verbindung mit einem Amin der Formel $R_5$—H, worin $R_5$ die vorstehend angegebene Bedeutung hat, und — sofern dies gewünscht wird — Umwandeln der erhaltenen Verbindung in ein pharmazeutisch annehmbares Säureadditionssalz in an sich bekannter Weise.

2. Verfahren nach Anspruch 1, dadurch gekennzeichnet, dass das genannte aprotische Lösungsmittel ausgewählt wird aus der Gruppe bestehend aus Tetrahydrofuran, Dimethylformamide und Dioxan.

3. Verfahren nach den Ansprüchen 1 oder 2, dadurch gekennzeichnet, dass die Reaktion der Verbindung der Formel (IX), wie sie in Anspruch 1 definiert ist, mit Ethylchloroformiat bei einer Temperatur von 0°C während 5 bis 6 h in Gegenwart einer organischen Base durchgeführt wird.

4. Verfahren nach Anspruch 3, dadurch gekennzeichnet, dass die organische Base Triethylamin darstellt.

5. Verfahren nach Anspruch 1, dadurch gekennzeichnet, dass die Kondensation bei Raumtemperatur während 2 bis 24 h ausgeführt wird.

**Revendications pour les Etats contractants: BE CH DE FR GB IT LI NL SE**

1. Dérivés d'ergoline ayant la formule (I)

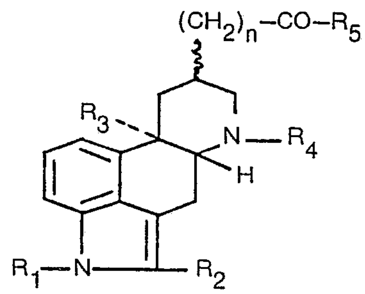

(I)

dans laquelle n est 0, 1 ou 2, $R_1$ représente un atome d'hydrogène ou un groupe méthyle, $R_2$ représente un atome d'hydrogène ou d'halogène ou un groupe méthyle, $R_3$ représente un atome d'hydrogène ou un groupe méthoxy, $R_4$ représente un groupe d'hydrocarbures ayant de 1 à 4 atomes de carbone, et $R_5$ est un rest des formules (II), (III), (IV), (V), (VI), (VII) ou (VIII)

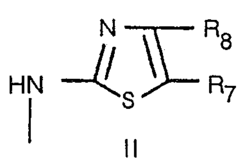

II

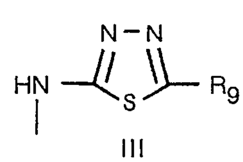

III

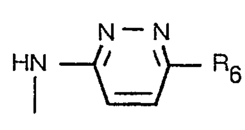

IV

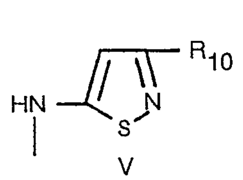

V

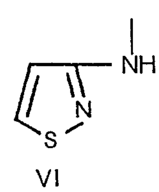

VI

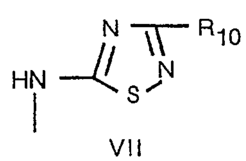

VII

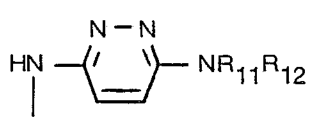

VIII

dans lesquelles $R_6$ représente un atome d'hydrogène ou de chlore ou un groupe méthoxy, et chacun de $R_7$, $R_8$ et $R_9$ représente indépendamment un atome d'hydrogène ou un groupe alkyle ayant de 1 à 3 atomes de carbone, avec la condition que lorsque $R_5$ représente un reste de formule générale (II) et que $R_2$, $R_7$ et $R_8$ représentent tous des atomes d'hydrogène, alors soit $R_4$ ne représente pas un groupe méthyle ou n n'est pas 0, ou un substituant sur $C_8$ n'est pas en configuration β et $R_{10}$ représente un atome d'hydrogène ou un groupe alkyle ayant de 1 à 3 atomes de carbone, soit chacun de $R_{11}$ et $R_{12}$ représente indépendamment un atome d'hydrogène, un groupe méthyle ou un groupe phényle, ou $R_{11}$ et $R_{12}$ forment ensemble avec l'atome d'azote auquel ils sont liés un anneau pyrrolidine, pipéridine, morpholine, pyrazole, imidazole ou pyrrole, et leurs sels pharmaceutiquement acceptables.

2. Composé selon la revendication 1 dans lequel l'anneau hétérocycle que $R_{11}$ et $R_{12}$ forment quand ils sont liés ensemble est choisi dans le groupe constitué par: pyrrolidine, pipéridine, morpholine, pyrazole, imidazole et pyrrole.

3. Composé selon la revendication 1 qui est choisi dans le groupe composé de:
6-méthyl-8β-(6-chloro-3-pyridazinyl-carbamoyl)-ergoline,
6-méthyl-8β-[2-(6-chloro-3-pyridazinyl-carbamoyl)-éthyl]-ergoline, et
6-méthyl-8β-(6-pyrrolyl-3-pyridazinyl-carbamoylméthyl)-ergoline.

4. Procédé pour préparer un dérivé d'ergoline conforme aux revendications 1, 2 ou 3, procédé qui consiste en ce qu'on fait réagir un composé de formule (IX)

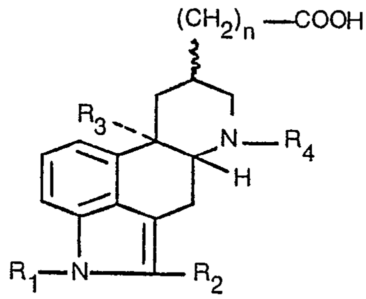

(IX)

dans laquelle n, $R_1$, $R_2$, $R_3$ et $R_4$ sont tels que définis dans la revendication 1, avec du chloroformate d'éthyle dans un solvant aprotique et on condense le composé résultant avec une amine de formule $R_5$—H, dans laquelle $R_5$ est tel que défini dans la revendication 1.

5. Procédé selon la revendication 4 selon lequel le solvant aprotique est choisi dans le groupe composé de: tétrahydrofuranne, diméthylformamide et dioxane.

6. Procédé selon les revendications 4 ou 5 selon lequel la réaction du composé de formule (IX) tel que défini dans la revendication 4 avec du chloroformate d'éthyle est exécutée à une température de 0°C pendant 5 à 6 heures en présence d'un base organique.

7. Procédé selon la revendication 6 selon lequel la base organique est la triéthylamine.

8. Procédé selon la revendication 4 selon lequel la condensation est exécutée à la température de la pièce pendant une durée de 2 à 24 heures.

9. Composition pharmaceutique contenant une quantité thérapeutiquement efficace d'un composé selon l'une quelconque des revendications 1 à 3, en mélange avec un porteur pharmaceutiquement acceptable.

10. Composition pharmaceutique selon la revendication 9 pour administration orale ou parentérale.

**Revendications pour l'Etat contractant: AT**

1. Procédé pour la préparation d'un dérivé d'ergoline ayant la formule (I)

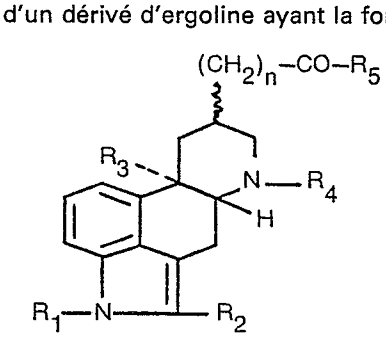

(I)

dans laquelle n est 0, 1 ou 2, $R_1$ représente un atome d'hydrogène ou un groupe méthyle, $R_2$ représente un atome d'hydrogène ou d'halogène ou un groupe méthyle, $R_3$ représente un atome d'hydrogène ou un groupe méthoxy, $R_4$ représente un groupe d'hydrocarbures ayant de 1 à 4 atomes de carbone, et $R_5$ est un reste des formules (II), (III), (IV), (V), (VI), (VII) ou (VIII)

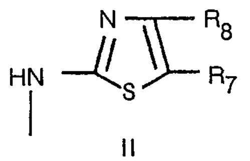
II

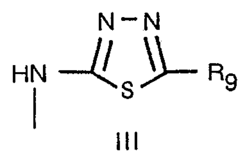
III

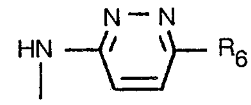
IV

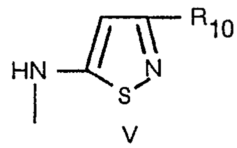
V

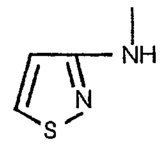
VI

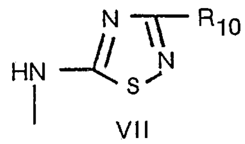
VII

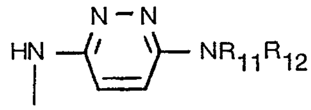
VIII

dans lesquelles $R_6$ représente un atome d'hydrogène ou de chlore ou un groupe méthoxy, et chacun de $R_7$, $R_8$ et $R_9$ représente indépendamment un atome d'hydrogène ou un groupe alkyle ayant de 1 à 3 atomes de carbone, avec la condition que lorsque $R_5$ représente un reste de formule générale (II) et que $R_2$, $R_7$ et $R_8$ représentent tous des atomes d'hydrogène, alors soit $R_4$ ne représente pas un groupe méthyle ou n n'est pas 0, ou un substituant sur $C_8$ n'est pas en configuration β et $R_{10}$ représente un atome d'hydrogène ou un groupe alkyle ayant de 1 à 3 atomes de carbone, soit chacun de $R_{11}$ et $R_{12}$ représente indépendamment un atome d'hydrogène, un groupe méthyle ou un groupe phényle, ou $R_{11}$ et $R_{12}$ forment ensemble avec l'atome d'azote auquel ils sont liés un anneau pyrrolidine, pipéridine, morpholine, pyrazole, imidazole ou pyrrole, et leurs sels pharmaceutiquement acceptables, procédé qui consiste en ce qu'on fait réagir un composé de formule (IX)

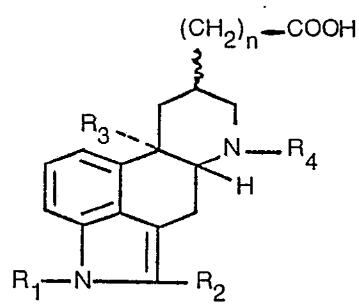
(IX)

dans laquelle n, $R_1$, $R_2$, $R_3$ et $R_4$ sont tels que définis ci-dessus, avec du chloroformate d'éthyle dans un solvant aprotique et on condense le composé résultant avec une amine de formule $R_5$—H, dans laquelle $R_5$ est tel que défini ci-dessus, et si nécessaire on convertit le composé résultant en un sel d'addition acide pharmaceutiquement acceptable, de manière connue en soi.

2. Procédé selon la revendication 1 selon lequel le solvant aprotique est choisi dans le groupe composé de: tétrahydrofuranne, diméthylformamide et dioxane.

3. Procédé selon les revendications 1 ou 2 selon lequel la réaction du composé de formule (IX) tel que défini dans la revendication 1, avec du chloroformate d'éthyle est exécutée à une température de 0°C pendant 5 à 6 heures en présence d'une base organique.

4. Procédé selon la revendication 3 selon lequel la base organique est la triéthylamine.

5. Procédé selon la revendication 1 selon lequel la condensation est exécutée à la température de la pièce pendant une durée de 2 à 24 heures.